(19)

Europäisches
Patentamt
European
Patent Office
Office européen
des brevets

(11) **EP 1 920 756 A1**

(12) **EUROPEAN PATENT APPLICATION**

(43) Date of publication:
**14.05.2008 Bulletin 2008/20**

(51) Int Cl.:
*A61K 8/34* (2006.01)   *A61K 8/37* (2006.01)
*A61K 8/44* (2006.01)   *A61K 8/67* (2006.01)
*A61K 8/92* (2006.01)   *A61K 8/97* (2006.01)
*A61Q 19/00* (2006.01)   *A61Q 19/08* (2006.01)

(21) Application number: **06425757.9**

(22) Date of filing: **07.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **SYRIO PHARMA S.P.A.**
**20129 Milano (IT)**

(72) Inventor: **Lazzeretti, Daniela**
**20138 Milano (IT)**

(74) Representative: **Perani, Aurelio et al**
**Perani Mezzanotte & Partners**
**Piazza San Babila, 5**
**20122 Milano (IT)**

(54) **Cosmetic matrix for a preservative-free cosmetic formulation**

(57)     The present invention relates to a water-based matrix for preparing preservative-free cosmetic preparations comprising: 2 to 40% by weight of at least one vegetable ester of glycerin with one or more mono- or polyunsaturated fatty acids, having 18 to 20 carbon atoms; 0.5 to 20% by weight of at least one non-triglyceride ester formed from an alcohol having 12 to 22 carbon atoms, and one or more saturated, monounsaturated and/or polyunsaturated fatty acids having 14 to 22 carbon atoms; 0.5 to 25% by weight of at least one polyol of functionality 2 to 12 and pentylene glycol; at least 0.012% by weight of at least one sequestrating agent; 0.2% to 20% by weight of at least one antioxidant agent; 0.0001% to 5% by weight of at least one unsaponifiable fraction of a natural oil and/or natural butter containing sterolic fractions of the natural plant from which it derives. Specifically, the invention relates to a preservative-free cosmetic preparation, comprising the matrix of the invention, in the form of a cream, preferably an anti-wrinkle cream for sensitive skins and a moisturizing cream for sensitive skins.

EP 1 920 756 A1

**Description**

The present invention relates to a new cosmetic matrix useful to make cosmetic products. Specifically, the invention relates to preservative-free cosmetic preparations for sensitive skins.

Several cosmetic formulations for the use on sensitive skin are commercially available. It is in fact known that sensitive or hyperactive skin is the one referable to undesired skin intolerances, atopic dermatitis, allergies and sensitisations. Subjects suffering from these types of reactions surely include normal skins, non pathological, but which can prove to be intolerant to a wide range of substances and external agents, such as environmental and atmospheric agents, as well as to be suffering from a stressful lifestyle or from excessive hygienic conditions.

Furthermore, such phenomena keep increasing and most of them are worsened or triggered by the use of cosmetic preparations. Evident skin consequences associable to these phenomena are high dehydration, severe skin dryness, alteration of the hydrolipidic film and pH, as well as loss of the barrier function, consequences worsening with aging.

Because of these issues, the most recent tendency in the development of cosmetic products is assuring not only the efficacy, but also the safety of use of the product for end users.

Among the many ingredients found in a cosmetic, one of the most discussed categories, precisely as for the toxicological profile, is that of preservative substances. It is anyway necessary to use such preservatives in order to protect cosmetic products against microbial pollution, which would compromise their safety of use.

A hard task for the cosmetic formulator is therefore to use the minimum amount of such preservatives while assuring the safety of use of the cosmetic itself. Specifically, the formulator who needs to reduce preservatives must keep into account factors causing micro-organisms to contaminate products, trying to limit both the contamination and proliferation of such micro-organisms. It is in fact possible to act on one or more of these factors by creating actual obstacles to factors favouring pollution and subsequent proliferation. It is anyway difficult to act on multiple factors to assure safety of use and concurrently not to modify the quality of the final cosmetic product both technically and as for its efficacy.

Therefore, the tendency is to always add preservatives in order to avoid contamination and proliferation of micro-organisms in finished products, but trying to limit the effects generated by preservatives themselves.

Creams for sensitive and delicate skins are known, which contain a certain amount of preservatives, but which try to prevent flushes or the tendency to irritation, although not pathological, by further adding substances which can restrict the action of free radicals.

It is therefore currently felt the need for cosmetic preparations for sensitive skins, for which, regardless of the cosmetic active ingredient used, it is possible to assure safety through time without using preservatives.

An object of the present invention is therefore to provide cosmetic preparations which are tolerable, safe and effective and which do not include preservatives.

The object mentioned above has been achieved through a water-based matrix for preparing preservative-free cosmetic preparations according to claim 1.

Specifically, the matrix according to the invention comprises:

-    2 to 40% by weight of at least one vegetable ester of glycerin with one or more mono- or polyunsaturated fatty acids, having 18 to 20 carbon atoms;
-    0.5 to 20% by weight of at least one non-triglyceride ester formed from an alcohol having 12 to 22 carbon atoms, and one or more saturated, monounsaturated and/or polyunsaturated fatty acids having 14 to 22 carbon atoms;
-    0.5 to 25% by weight of at least one polyol of functionality 2 to 12 and pentylene glycol;
-    at least 0.012% by weight of at least one sequestrating agent;
-    0.2% to 20% by weight of at least one antioxidant agent;
-    0.0001% to 5% by weight of at least one unsaponifiable fraction of a natural oil and/or natural butter containing sterolic fractions of the natural plant from which it derives.

As used in the present invention, the following term:

"water-based matrix" means a basal formulation for a cosmetic preparation in the water solvent having cosmetic quality;
"cosmetic active ingredient" means one or more cosmetic grade active principles to be added to the matrix of the invention in order to obtain the final cosmetic preparation having desired cosmetic properties. For example, in order to prepare anti-wrinkle cosmetic preparations, it will be possible to add to the matrix of the invention all the substances known for having an anti-wrinkle cosmetic property.

When amounts are indicated in the description, they are expressed as percentages by weight with respect to the total weight of the ingredients. Therefore, when the matrix and cosmetic formulation amounts are given, they will be expressed as percentages by weight with respect to the weight of the total matrix and the cosmetic formulation, respectively.

Further advantages and characteristics of the matrix according to the invention are recited in the dependent claims.

The invention will be now detailed with reference to the attached drawings for not limitative and exemplification purposes, in which:

Figure 1 a is a graph of cell viability results (%) of example 5 after treatment with increasing doses of Sodium Lauryl Sulphate;
Figure 1b is a graph of cell viability results (%) of example 5 after treatment with increasing doses of moisturizing cream for sensitive skins L9V9;
figure 2a is a graph of cell viability results (%) of example 7 after treatment with increasing doses of Sodium Lauryl Sulphate;
Figure 2b is a graph of cell viability results (%) of example 7 after treatment with increasing doses of anti-wrinkle cream for sensitive skins L10V10;
Figure 3a is a graph of cell viability results (%) of example 9 after treatment with increasing doses of Sodium Lauryl Sulphate;
Figure 3b is a graph of cell viability results (%) of example 9 after treatment with increasing doses of moisturizing cream for sensitive skins L11V11;
Figure 4a is a graph of cell viability results (%) of example 11 after treatment with increasing doses of Sodium Lauryl Sulphate; and
Figure 4b is a graph of cell viability results (%) of example 11 after treatment with increasing doses of anti-wrinkle cream for sensitive skins L12V12.

The matrix according to the invention comprises at least one vegetable ester of glycerin with one or more mono- and/or polyunsaturated fatty acids, having from 18 to 20 carbon atoms. Such ester is preferably selected from the group consisting of sweet almond oil, wheat germ oil, olive oil, avocado oil, argan oil, soybean oil, rapeseed oil, corn oil, rice oil, borage oil, macadamia oil, evening primerose oil (oenothera oil), rose hip seed oil (rose mosqueta oil), soybean glycerides, shea butter (karate butter), crab oil, hazelnut butter, illipe butter and mixtures thereof, more preferably it is selected from the group consisting of soybean oil, wheat germ oil, avocado oil and mixtures thereof. According to the invention, the ester amount will range from 2 to 40% by weight, preferably from 3 to 20% by weight, more preferably from 5 to 15% by weight.

The at least one non-triglyceride ester formed from an alcohol having 12 to 22 carbon atoms, and one or more saturated, monounsaturated and/or polyunsaturated fatty acids having 14 to 22 carbon atoms, is preferably selected from the group consisting of cetearyl olivate, sorbitan olivate, jojoba wax, glyceryl stearate and mixtures thereof, more preferably it is selected from cetearyl olivate, sorbitan olivate and mixtures thereof. Such a non-triglyceride ester is present in the matrix according to the invention in amounts ranging from 0.5 to 20% by weight, preferably from 1 to 10% by weight, more preferably from 1 to 6% by weight.

The polyols of the matrix of functionality 2 to 12 according to the invention will preferably be selected from the group consisting of glycols, polyalcohols, saccharides and polysaccharides. More preferably, they will be selected from the group consisting of glycerin, sorbitol and mixtures thereof. They will preferably be in amounts ranging from 2 to 6%.

The at least one sequestrating agent can be any agent able to sequestrate metal ions through the formation of a complex. It will preferably be ethylendiaminetetraacetic acid and/or salts thereof with alkaline metals, particularly its mono-, bi-, tri- or tetra-sodium salts. It will be preferably present in amounts of about 0.1 % by weight.

The matrix according to the invention comprises at least one antioxidant agent in amounts ranging from 0.5 to 20% by weight. The antioxidant agent is preferably selected from the group consisting of alphatocopherol and its esters, BHA, esters of gallic acid, nordihydroguaiaretic acid, idebenone, flavonoids, ascorbic acid, salts and esters of ascorbic acid, carotenoids, tocotrienols and pycnogenol. More preferably it is selected from the group consisting of tocopheryl acetate and ascorbyl palmitate and mixtures thereof. Advantegeously, the at least one antioxidant agent is present in amounts of about 3-4%.

Together with the above-mentioned ingredients, it is also provided at least one unsaponifiable fraction of vegetable oils and butters, such as for example shea butter (karite butter), soybean oil, olive oil, wheat germ oil, containing the sterolic fraction of the plant from which it derives, being sterolic fractions for example, sitosterols, cholesterol and stigmasterols.

The matrix of the invention also comprises pentylene glycol, preferably ranging from 3 to 6%, and more preferably is about 4%.

Being the matrix of the invention water-based, it will be added with water in amounts sufficient to achieve 100% of the final matrix. Water according to the invention will preferably be water previously deionized by means of an inverted osmosis apparatus.

Therefore, one or more active cosmetic ingredients useful to form the desired cosmetic formulation are added to the matrix according to the invention.

In another aspect, the invention relates to a preservative-free cosmetic formulation comprising the matrix of the invention and one or more cosmetic active ingredients.

The cosmetic formulation comprising the matrix according to the invention and one or more cosmetic active ingredients comprises the ingredients of the matrix itself in the same amounts as the matrix mentioned above, being the ingredient water present in such an amount as to reach 100% by weight of the final cosmetic formulation.

In a first embodiment of the cosmetic formulation according to the invention, the matrix according to the invention is added with cosmetic active ingredients such as to form an anti-wrinkle cosmetic formulation. Preferably, the anti-wrinkle formulation comprises an amount not lower than 1% of cosmetic active ingredients with respect to the weight of the final cosmetic formulation, preferably 1 to 10%. Such cosmetic active ingredients are advantageously one or more cosmetic active ingredients selected from the group consisting of glycerin, hyaluronic acid and its derivatives, retinol and its derivatives, ascorbyl palmitate and tocopheryl acetate, phospholipids and mixtures thereof. As the matrix according to the invention comprises antioxidant agents which can be anti-wrinkle cosmetic active principles, the antioxidant agents of the matrix, if cosmetic active principles, will also help in reaching the minimum amount of the anti-wrinkle cosmetic active principles.

In a second embodiment of the cosmetic formulation according to the invention, the matrix according to the invention is added with cosmetic active ingredients such as to form a moisturizing cosmetic formulation. Preferably, the moisturizing formulation comprises an amount not lower than 0.5% of the moisturizing cosmetic active ingredients with respect to the weight of the final formulation, preferably 0.5 to 10%. Such cosmetic active ingredients are advantageously one or more cosmetic active ingredients selected from the group consisting of glycerin, hyaluronic acid and its derivatives and mixtures thereof.

The cosmetic formulation comprising the matrix and suitable cosmetic active ingredients can also comprise appropriate cosmetic grade eccipients with functionality of rheology modifiers, blockers of free water, emulsion stabilizers.

Cosmetic grade excipients which can be added to the composition according to the invention comprise polymeric substances such as for example, acrylates, cellulose and its derivatives, vinylderivatives, polysaccharides, natural proteic substances and/or their derivatives and hydrolyzed.

Such excipients are present into the final formulation in amounts ranging from 0.003% to 15% with respect to the final weight of the cosmetic formulation.

The preservative-free cosmetic formulations can be in the form of, for example, a cream, a gel, a lypogel, or a lotion.

It will be therefore possible to use all known processes for preparing a cream, a gel, a lypogel, or a lotion of the preservative-free cosmetic formulation according to the invention comprising the water-based matrix, one or more cosmetic active ingredients and cosmetic grade excipients with functionality of rheology modifiers, blockers of free water, emulsion stabilizers.

Preferably the formulation according to the invention is in the form of a cream.

Therefore, in another aspect, the invention relates to a process of preparing a cream having the formulation according to the invention.

Specifically, the process of preparing a cream having the formulation of the invention, comprises the steps of:

a) preparing an aqueous phase and an oil phase with ingredients selected from:

- at least one polyol of functionality 2 to 12;
- at least one sequestrating agent,
- pentylene glycol,
- at least one non-triglyceride ester formed from an alcohol having 12 to 22 carbon atoms, and one or more saturated, monounsaturated and/or polyunsaturated fatty acids having 14 to 22 carbon atoms;
- at least one antioxidant agent,
- at least one unsaponifiable fraction of a natural oil and/or natural butter containing sterolic fractions of the natural plant from which it derives and
- optionally excipients,

wherein each ingredient is comprised either in the aqueous phase or in the oil phase based on its nature;
b) bringing about 50% of the aqueous phase and all the oil phase to a temperature of about 40°C and mixing them until an emulsion is produced,
c) bringing the resulting emulsion to about 25°C and adding the remaining 50% of the aqueous phase and at least one vegetable ester of glycerin with one or more mono- or polyunsaturated fatty acids, having 18 to 20 carbon atoms;
d) pasteurizing the resulting emulsion at a temperature ranging from 58°C to 62°C; and
e) pouring the pasteurized emulsion into sterile containers;

wherein in either the aqueous phase or the oil phase o in both phases one or more cosmetic active ingredients are present.
Both the aqueous phase and the oil phase respectively of the step, according to the invention, are prepared separately, concurrently or consecutively by using appropriate containers and measuring the amounts of raw materials to be used preferably by means of a liter counter for water and a balance for the other components, regardless if they are solids or liquids.
50% of the aqueous phase of step b) which must be brought to a temperature of about 40°C is preferably loaded in a turboemulsifier equipped with temperature and stirring intensity regulation systems. The efficacy of the latter is assured by rotating counterblades and suspended turbine system. The aqueous phase contained into the turboemulsifier is then brought to a temperature of 40°C, preferably under weak stirring, that is in the turboemulsifier, with the turbine still and the blades rotating at a set speed of 20 RPM.
Preferably, the oil phase is prepared to the emulsifying process of step b) by using a "melter", i.e. a containment system equipped with temperature regulation and mechanical stirring. The oil phase of the product of interest is thus brought to a temperature of 40°C, at which it has to be perfectly homogeneous.
In step b), when 50% of the aqueous phase and all the oil phase, contained, preferably and respectively in the turboemulsifier and in the melter, reach the temperature of 40°C, the oil phase is added to the acqueous phase under stirring, by using a suitable piping allowing the transfer of the melter content to the turboemulsifier. The stirring intensity is advantegeously adjusted by setting the rotational speed of the turboemulsifier blades at 30 RPM and that of the turbine at 1000 RPM.
This stirring speed is preferably maintained (always at 40 °C) for about 45 minutes, in order to allow the dispersion of the oil phase in the aqueous phase, i.e. the emulsification.
In step c), the so formed emulsion is then cooled to 25 °C, by advantageously setting a cooling speed of 0.1 °C/min. During cooling, the speed of the turboemulsifier blades is preferably increased to 50 RPM, while the turbine is stopped. Once the temperature of 25°C is reached, the rotational speed of the blades is then preferably decreased to 30 RPM, while the turbine is started again and brought to 2000 RPM.
In step c), the remaining 50% of aqueous phase and one or more esters, in the meantime advantageously maintained, each in a separate container, at room temperature (about 25°C), are then added.
Before the pasteurizing step d), the emulsion of step c) is advantegeously brought to a temperature of about 30 °C by setting a heating temperature of 0.067 °C/min, and once it is reached, the thermoemulsifier turbine is preferably stopped, while the movement of the blades is unchanged.
In step d), the turboemulsifier is then advantageously connected to a pasteurizer, which will preferably be a plate pasteurizer and the emulsion is pasteurized at a temperature ranging from 58°C to 62°C, preferably 60 °C.
When outing the pasteurization step d), the product is fed into sterile containers.
The invention will be now described with reference to some examples of preparing the formulations, given by way of illustration and not limiting the invention, and to evaluation tests showing the results yielded by the invention.

**Example 1**

Preparation of moisturizing cream formulation for sensitive skins, referred to as L9V9:

An aqueous phase comprising sorbytol (as polyol), disodium EDTA (as sequestrating agent), tocopheryl acetate and ascorbyl palmitate (as antioxidants), pentylene glycol and deionized water and an oil phase comprising cetearyl olivate, sorbitan olivate (as non-triglyceride esters), unsaponifiable fraction of shea butter (Butyrospermum parkii) (as unsaponifiable fraction of the butter) were prepared.
In separate containers, the following vegetable esters were prepared: wheat germ oil, avocado oil, soybean glycerides.
Glycerin and sodium hyaluronate were used as cosmetic active ingredients, and were added to the aqueous phase.
50% of the aqueous phase was loaded into a turboemulsifier equipped with temperature and stirring intensity regulation systems. The aqueous phase contained in the turboemulsifier was then brought to a temperature of 40°C, under weak stirring, with the turbine still and a rotational speed of the blades set at 20 RPM.
The oil phase was prepared using a melter and was brought to a temperature of 40°C, at which temperature it was perfectly homogeneous.
When 50% of the aqueous phase and all the oil phase, contained, preferably and respectively, in the turboemulsifier and in the melter, reached the temperature of 40°C, the oil phase was added to the acqueous phase under stirring, by using a suitable piping allowing the transfer of the melter content to the turboemulsifier. The stirring intensity was adjusted by setting the rotational speed of the turboemulsifier blades at 30 RPM and that of the turbine at 1000 RPM. This stirring speed at 40°C was maintained for about 45 minutes, in order to allow the dispersion of the oil phase in the aqueous phase, i.e. the emulsification.

An emulsion was thus prepared, which was then cooled to 25°C by advantageously setting a cooling speed of 0.1 °C/min. During cooling, the speed of the turboemulsifier blades was increased to 50 RPM, while the turbine was stopped. Once the temperature of 25°C was reached, the rotational speed of the blades was then decreased to 30 RPM, while the turbine was started again and brought to 2000 RPM. In such stirring conditions, the remaining 50% of aqueous phase was then added and wheat germ oil, avocado oil and soyabean glycerides, maintained in separate containers, were added at 25°C.

The emulsion was then brought to a temperature of about 30°C by setting a heating temperature of 0.067 °C/min, and once this temperature was reached, the termoemulsifier turbine was stopped, while the movement of the blades was unchanged. The turboemulsifier was them connected to a plate pasteurizer and the emulsion was pasteurized at 60 °C. The emulsion in the form of a cream was then fed into sterile containers.

The formulation of the conditioning cream for sensitive skins L9V9 was the following:

| Matrix of the invention: | |
| --- | --- |
| sorbytol | 3.0% |
| disodium EDTA | 0.1% |
| pentylene glycol | 4.0% |
| cetearyl olivate | 2.7% |
| sorbitan olivate | 1.8% |
| tocopheryl acetate | 0.3% |
| ascorbyl palmitate | 0.0015% |
| Butyrospermum parkii fraction | 0.45% |
| wheat germ oil | 6.0% |
| avocado oil | 5.0% |
| soybean glicerydes | 1.0485% |
| Cosmetic active ingredients: | |
| glycerin | 2.0% |
| sodium hyaluronate | 0.0080% |
| and deionized water | q.b. at 100% |

**Example 2**

Preparation of anti-wrinkle cream formulation for sensitive skins, referred to as L10V10:

By following the process of example 1 the formulation L10V10 was prepared using the ingredients set forth below.

An aqueous phase comprising sorbytol (as polyol), disodium EDTA (as sequestrating agent), tocopheryl acetate and ascorbyl palmitate (as antioxidants), pentylene glycol and deionized water and an oil phase comprising cetearyl olivate, sorbitan olivate (as non-triglyceride esters), unsaponifiable fraction of shea butter (Butyrospermum parkii) (as unsaponifiable fraction of the butter) were prepared.

In separate containers, the following vegetable esters were prepared: wheat germ oil, avocado oil, soybean glycerides.

Phospholipids which were added respectively to the oil phase, and glycerin, retinyl palmitate and sodium hyaluronate which were added to the aqueous phase, were employed as anti-wrinkle cosmetic active ingredients. Also tocopheryl acetate as antioxidant of the aqueous phase served as anti-wrinkle cosmetic active ingredient.

The resulting formulation of the anti-wrinkle cream for sensitive skins L10V10 was the following:

| Matrix of the invention: | |
| --- | --- |
| sorbytol | 3.0% |
| disodium EDTA | 0.1% |
| pentylene glycol | 4.0% |
| cetearyl olivate | 2.7% |

(continued)

| Matrix of the invention: | |
|---|---|
| sorbitan olivate | 1.8% |
| tocopheryl acetate | 0.3% |
| ascorbyl palmitate | 0.0027% |
| Butyrospermum parkii fraction | 0.45% |
| wheat germ oil | 6.0% |
| avocado oil | 5.0% |
| soybean glicerydes | 1.0485% |
| Cosmetic active ingredients: | |
| glycerin: | 1.0% |
| sodium hyaluronate: | 0.010% |
| retinyl palmitate: | 0.3% |
| phospholipids: | 0.01 % |
| and deionized water: | q.b. at 100% |

**Example 3**

Preparation of moisturizing cream formulation for sensitive skins, referred to as L11V11:

By following the process of example 1, the preparation L11 V 11 was prepared using the ingredients set forth below.

An aqueous phase comprising glycerin (as polyol); disodium EDTA (as sequestrating agent), tocopheryl acetate (as antioxidant), pentylene glycol and deionized water and an oil phase comprising glyceryl stearate, (as non-triglyceride ester), unsaponifiable fraction of olive oil (olea europaea) (as unsaponifiable fraction) were prepared.

In a separate container, the following vegetable ester was prepared:

soybean oil.

Sodium hyaluranate and glycerin were used as moisturizing cosmetic active ingredients, being the latter already present in the formulation as polyol, and they were added to the aqueous phase.

Ammonium acryloyldimethyltaurate/VP copolymer was also added to the aqueous phase as additive able to increase viscosity.

The resulting formulation of the moisturizing cream for sensitive skins L11V11 was the following:

| Matrix of the invention: | |
|---|---|
| glycerin | 3.0% |
| disodium EDTA | 0.1% |
| pentylene glycol | 4.0% |
| glyceryl stearate | 3.0% |
| tocopheryl acetate | 0.3% |
| olive oil fraction | 1.00% |
| soybean oil | 10.0% |
| Cosmetic active ingredient: | |
| sodium hyaluronate | 0.0080% |
| Additive: | |
| ammonium acryloyldimethyltaurate/VP copolymer | 0.6% |
| and deionized water: | q.b. at 100% |

**Example 4**

Preparation of anti-wrinkle cream formulation for sensitive skins, referred to as L12V12:

By following the process of example 1, the preparation L12V12 was prepared using the ingredients set forth below.

An aqueous phase comprising glycerin (as polyol); disodium EDTA (as sequestrating agent), tocopheryl acetate and ascorbyl palmitate (as antioxidants), pentylene glycol and deionized water and an oil phase comprising glyceryl stearate, (as non-triglyceride ester), unsaponifiable fraction of olive oil (olea europaea) (as unsaponifiable fraction) were prepared.

In a separate container, the following vegetable ester was prepared: soybean oil.

Phospholipids which were added to the oil phase, and sodium hyaluronate and retinyl palmitate which were added to the aqueous phase were employed as anti-wrinkle cosmetic active ingredients. Also tocopheryl acetate as antioxidant and glycerin of the aqueous phase served as anti-wrinkle cosmetic active ingredients.

Ammonium acryloyldimethyltaurate/VP copolymer was also added to the aqueous phase as additive able to increase viscosity.

The resulting formulation of the anti-wrinkle cream for sensitive skins was the following:

Matrix of the invention:

| | |
|---|---|
| glycerin | 3.0% |
| disodium EDTA | 0.1% |
| pentylene glycol | 4.0% |
| glyceryl stearate | 3.0% |
| tocopheryl acetate | 0.3% |
| ascorbyl palmitate | 0.0012% |
| unsaponifiable fraction | 1.00% |
| soybean oil | 10.0% |
| Cosmetic active ingredients: | |
| sodium hyaluronate | 0.010% |
| retinyl palmitate | 0.3% |
| phospholipids | 0.01 % |
| Additive: | |
| ammonium acryloyldimethyltaurate/VP copolymer | 0.6% |
| and deionized water: | q.b. at 100% |

**Example 5**

Cytotoxicity evaluation of moisturizing cream L9V9 with MTT method (UNI EN ISO 10993-5 rule)

The test consisted in evaluating the cytotoxicity of the moisturizing cream L9V9 aimed to be used on skin or on mucosae following the UNI EN ISO 10993-5 rule concerning the biological evaluation of medical devices.

The cytotoxicity test was carried out towards the keratinocyte, the main cell component of skin and mucosae.

The method used was the MTT method, originally developed by Mossmann, which implies the use of the reagent 3-[4,5-dimethylthiazol-2-yl]-2,5-diphenyl tetrazolium bromide or MTT, which is of yellowish colour in aqueous solution. The method is based on the fact that the mitochondrial dehydrogenases of viable cells are able to cleave the tetrazolium ring of MTT, leading to the formation of purple formazane crystals, which are insoluble in water. The crystals are dissolved in acidified isopropanol and the resulting purple solution is measured spectrophotometrically. An increase or decrease in viable cell number results in a concomitant change in the amount of formazan which is forming and can be considered as an indication of the degree of cytotoxicity caused by the exposure to the tested substances.

The cell model employed for the in vitro test consisted of stabilized human keratinocytes (HaCaT cells).

Cells were cultured in 24-well plates for 24 hours in DMEM + 10% FCS. Fresh culture medium was then added, supplemented with 10% FCS and containing a sample of the product to be tested so as to reach 6 final dilutions ranging from 5 to 0.15 mg/ml.

For each sample of the cream L9V9, three replica were performed.

Untreated cells were used as negative control, and as positive control cells were treated with a surfactant with known toxicity, i.e. Sodium Lauryl Sulphate briefly referred to as SLS, dissolved in the culture medium in concentrations ranging from 0.5 mg/ml to 0.03 mg/ml. Specifically, samples with SLS amounts equal to 0.5, 0.25, 0.12, 0.06 and 0.03 mg/ml and L9V9 amounts of 5, 2.5, 1.25, 0.6, 0.3, 0.15 mg/ml were prepared.

At the end of treatment, the citotoxicity test (MTT) was carried out. The cells were then washed with washing solution (Dulbecco's Phosphate Buffered Saline).

After removal of the solution, the MTT-culture medium was added to each well and the cells were incubated at 37°C.

At the end of the incubation period, the MTT-culture medium was removed and a MTT solubilizing solution was added to each well. The plate was then stirred on a plate stirrer, while ensuring that all the crystals were dissolved and had formed a homogeneous solution.

The absorbance was then read with a chromatometer equipped with a plate reader, by subtracting background reading. The result was expressed as:

$$\% \text{ cell survival} = \frac{\text{OD treated cells x 100}}{\text{OD untreated cells}}$$

The results obtained for SLS and L9V9 were as set forth in Table 1 below:

**Table 1: cell viability (%) for SLS and cream L9V9**

| SLS (positive control) | | | | | | |
|---|---|---|---|---|---|---|
| Dose mg/ml | | 0.5 | 0.25 | 0.12 | 0.06 | 0.03 |
| Cell viability % (vs negative control) | | 2.3 | 2.3 | 2.5 | 61.0 | 87.6 |
| Stand. dev. | | 0.9 | 0.7 | 1.1 | 4.1 | 4.6 |
| Moisturizing cream for sensitive skins L9V9 | | | | | | |
| Dose mg/ml | 5 | 2.5 | 1.25 | 0.6 | 0.3 | 0.15 |
| Cell viability % (vs negative control) | 92.4 | 96.7 | 108.5 | 114.0 | 111.9 | 106.5 |
| Stand. dev. | 1.9 | 2.8 | 4.4 | 6.8 | 4.9 | 4.8 |

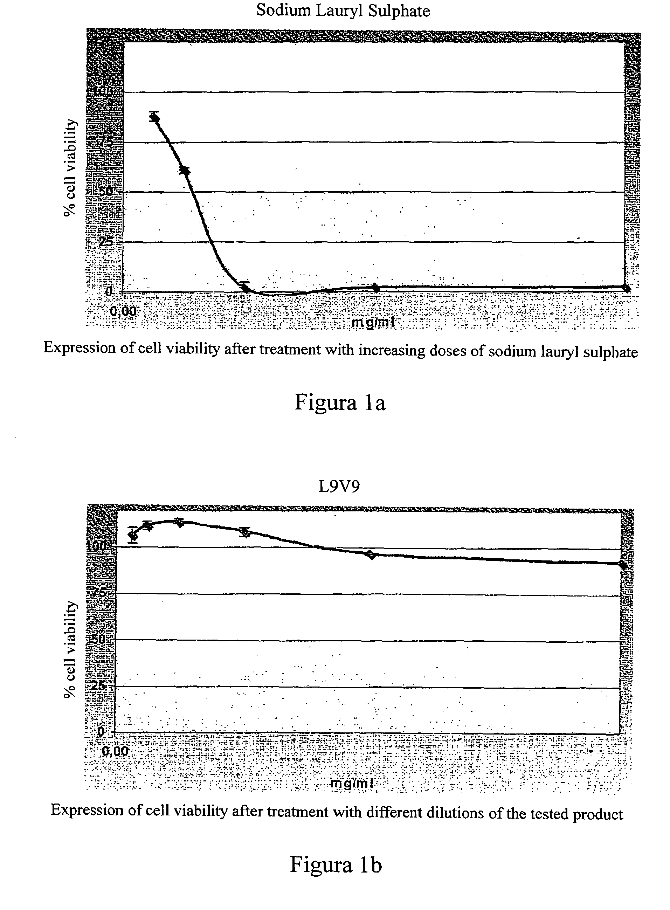

The cytotoxicity data obtained with the MTT test, corrected by subtracting the absorbance readings due to the diluent medium, were plotted against the concentration of the tested product, thus generating a dose-response curve, shown in Figure 1a for SLS and 1b for L9V9, which allowed to determine the theoretical regression curve for evaluating the concentration of 50% inhibition of cell growth ($IC_{50}$).

The theoretical value of the concentration of 50% inhibition of cell growth ($IC_{50}$) was thus obtained, i.e the concentration which induces a decrease in cell viability by 50% in compare to untreated cells. Such an $IC_{50}$ value was the parameter which allowed the irritating potential of the cream to be evaluated, wherein values < 1 mg/ml were deemed irritating, values > 3 mg/ml showed an optimal biocompatibility and values ranging from 1 to 3 indicated a discrete biocompatibility.

The $IC_{50}$ value for SLS was 0.06 mg/ml, while the one for L9V9 was $IC_{50}$> 5 mg/ml.

On basis of the above mentioned results, the product moisturizing cream for sensitive skins L9V9 did not show any cytotoxic effects on human keratinocytes. These data are thus deemed predictive of absence of irritating effects in vivo.

**Example 6**

<u>In vitro analysis of the pro-sensitising potential of moisturizing_ cream for sensitive skins L9V9</u>

The purpose of the test was to evaluate that the absence of pro-sensitising effects in cream L9V9. The test consisted in evaluating the pro-sensitising stimulus on human monocytes. It was deemed appropriate to use a cell model to evaluate the immunological reactivity of typical immunological cells (monocytes in this case) exposed for a prolonged time (48h) to the tested product in two different concentrations

The test was thus performed on a monocytes cell line named THP-1, as such a cell type is highly involved in the immunological responses of skin, which is the target organ of the tested cream. Therefore, on these cells the expression modulation of two co-stimulatory molecules, CD80 (B7.1) and CD86 (B7.2), was evaluated, using as a positive control Nickel Sulphate ($NiSO_4 6H_2O$), a typical contact sensitising substance, specifically in crystals dissolved in PBS. Nickel is in fact able to trigger in vivo allergising-type immunological reactions (contact sensitising).

The technique is based on the fact that the recognition of the antigen by the TCR (T Cell Receptor) of T lymphocytes (signal 1) is not functional to the maturation of an effective immune response, if it doesn't occur at the membrane of an antigen-presenting cell able to provide an additional signal (signal 2 or costimulus) qualitatively necessary for the definition of the response type (humoral, cellular, etc). Both B7.1 and B7.2 (collectively: B7) are membrane glycoproteins which are present on the surface of several antigen-presenting cells (dendritic cells, Langerhans cells, monocytes/macrophages, other cell lines such as keratinocytes) and serve as costimula. In fact, both these molecules are ligands of a glycoprotein named CD28, which are present on the T lymphocyte membrane. The triggering of the ligand/receptor system CD28B7 avoids the T cell apoptosis (i.e. the programmed cell death) and cooperates to sustain their proliferation and differentiation. In the first steps of the "physiological" immune response, B7.2 is expressed constitutively and modulates both the Th1 and Th2 responses. As the immune response goes on, also B7.1 is up-regulated and increases the intensity of the co-stomulatory signal, with an expansion of T cells and production of many citokines. Furthermore, B7.1 is preferably up-regulated during the acute phase of auto-immune responses.

An increase in expression of these co-stimulatory molecules on monocytes is thus a signal of activation of an immune response the exposure in consequence of a potentially allergising antigen. Functionally, in fact, the expression of co-stimulatory molecules on this cell type corresponds to the competence acquisition for expressing the antigen in the typical location (skin in this case) in which a contact sensitization occurs in vivo.

A sample of the cream L9V9 and a sample of nickel sulphate were dissolved in ethanol and then diluted directly in the cell culture medium at different dilutions. They were subjected to a preliminary cytotoxicity screening on THP-1 cells to determinate the concentration at which the substance could be used in vitro without causing cell death, event which causes false results.

Cell growth culture medium exposed to the same experimental conditions was used as a negative control.

The monocyte-like human line cell, named THP-1, was cultivated in RPMI containing 10% FCS and 2 mM glutamine. The MTT test was used as preliminary test. Therefore, by following the method of the example of the MTT test, the concentrations of L9V9 and nickel sulphate to be used were obtained.

L9V9 sample was employed at two different final dilutions on THP-1 cells, by diluting it in the cell culture medium so as to obtain the desired final concentrations in contact with the cells. The cells were exposed for 48 h at 37°C with 5% $CO_2$.

After exposure, the cells were examined for their viability by staining with Trypan Blue dye and by observing under microscope in a corpuscle-counter chamber, collected, washed in a isotonic buffer (PBS) and then marked with a fluoresceinated anti-B7.1 or B7.2 antibody. After further washings to remove the excess antibody, the cells were feeded in a flux cytofluorimeter (FACS, Fluorescence Activated Cell Sorter, Becton Dickinson, Mountain View, CA) for the MFI (Mean Fluorescence Intensity) evaluation, which was proportional to the number of molecules marked per cell and therefore representative of the expression level of the tested co-stimulatory molecules.

Morphological qualitative parameters (cell volume change, modifications in the cellular granulations) linked to cellular necrosis and apoptosis were also detected, events strictly linked to the allergic elicitation process.

As control (basal fluorescence), the MFI of both untreated THP-1 cells (apart from washings in PBS) and THP-1 cells reacted with a fluoresceinated monoclonal antibody (such as the anti-B7.1 and anti-B7.2), but of irrelevant specificity (isotype-matched control) was evaluated.

Table 2 below shows the results of the analysis of the monocyte cell line THP-1 for expression of co-stimulatory molecules at flux cytofluorimeter after 48 hours of reaction with the samples at the two tested concentrations and with the controls, corrected by the negative control.

Table 2

| Samples | CD80 (MFI*) | CD86 (MFI*) |
|---|---|---|
| Nickel sulphate 20 μg/ml | 29.00 | 77.12 |
| Nickel sulphate 10 μg/ml | 19.85 | 37.35 |
| Nickel sulphate 4 μg/ml | 0.41 | 0.79 |
| moisturizing cream for sensitive skins L9V9 5.0 mg/ml | -0.05 | 0.00 |
| moisturizing cream for sensitive skins L9V9 1 mg/ml | -0.06 | -0.12 |
| *MFI = Mean Fluorescente Intensity, i.e. the geometric average of the fluorescence intensity of the cells stained with the fluoresceinated antibody and it is proportional to the number of stained molecules per cell. | | |

By observing the behaviour of Nickel, a typical allergising substance, it was seen how this substance was characterised by a) a high increase of both markers; b) direct correlation between the increase of intensity of the response and the concentration; c) detactable effect even at very low doses.

The tested dose of 4 μg/ml of Nickel Sulphate (NiSO$_4$ 6H$_2$O) corresponded to about 1 ppm of Nickel, a value that was around the allergising threshold in already sensitised individuals and on irritated skin (Gawkrodger DJ, Nickel dermatitis: how much Nickel is safe? Contact Dermatitis 1996; 35:267-271; Tanojo H, Hostynek JJ, Mountford HB, Mibach HI, In vitro premeation of nickel salts through human stratum corneum. Acta Derm Venereol Suppl, 2001; 212: 19-23). In the test it was verified how this concentration was able of causing a detectable increase in CD80, with respect to untreated controls (data not shown).

The concentration able to cause an allergic reaction in most of the sensitive subjects was anyway around much higher values, above 100 ppm of Nickel Sulphate 6H$_2$O, in contact with healthy and intact skin.

At the tested concentrations, cream L9V9 had not shown any modulation of the investigated markers.

Cream L9V9 had not shown any cytotoxic effects on the cells used for the test (IC$_{50}$= 25 mg/ml). At the used dilutions, no apoptoptic effects on the examined cells came out.

In the above test the finished cosmetic product moisturizing cream for sensitive skins L9V9 didn't increase expression of the investigated markers in human monocytes in this *in vitro* model, thus showing that it does not have any stimulatory potential on the immune system mediated by monocyte/macrophage.

**Example 7**

Cytotoxicity evaluation of anti-wrinkle cream L10V10 with MTT method

The cytotoxicity evaluation test of the moisturizing cream for sensitive skins of example 5 was repeated, but using samples of anti-wrinkle cream for sensitive skins L10V10 as prepared in example 2.

Specifically, the same cell type (stabilized human keratinocytes (HaCaT cells)), the same equipment for the evaluation, the same culture medium, and the same amounts of cream were employed, and the same method was performed as in example 5. Untreated cells were used as negative control, while as positive control cells were treated with Sodium Lauryl Sulphate (SLS), dissolved in the culture medium at the same concentrations as in example 5 (0.5, 0.25, 0.12, 0.06 and 0.03 mg/ml).

At the end of the incubation period of each well, MTT-culture medium was removed and a MTT solubilizing solution was added to each well as in example 5.

The plate was then stirred on a plate stirrer, ensuring that all the crystals were dissolved and had formed a homogeneous solution.

The absorbance was then read with a chromatometer equipped with a plate reader by subtracting background reading, thus obtaining the percentage of cell survival.

The results obtained for SLS and L10V10 were as set forth in Table 3 below.

**Table 3: cell viability (%) for SLS and cream L10V10**

| SLS (positive control) | | | | | |
|---|---|---|---|---|---|
| Dose mg/ml | 0.5 | 0.25 | 0.12 | 0.06 | 0.03 |
| Cell viability % (vs negative control) | 2.2 | 2.9 | 3.9 | 79.6 | 94.7 |
| Stand. dev. | 0.8 | 0.6 | 0.6 | 8.4 | 7.7 |
| Anti-wrinkle cream for sensitive skins L10V10 | | | | | |
| Dose mg/ml | 5 | 2.5 | 1.25 | 0.6 | 0.3 | 0.15 |
| Cell viability % (vs negative control) | 92.8 | 100.6 | 105.2 | 111.6 | 110.1 | 108.0 |
| Stand. dev. | 2.7 | 4.5 | 6.7 | 7.4 | 5.6 | 2.6 |

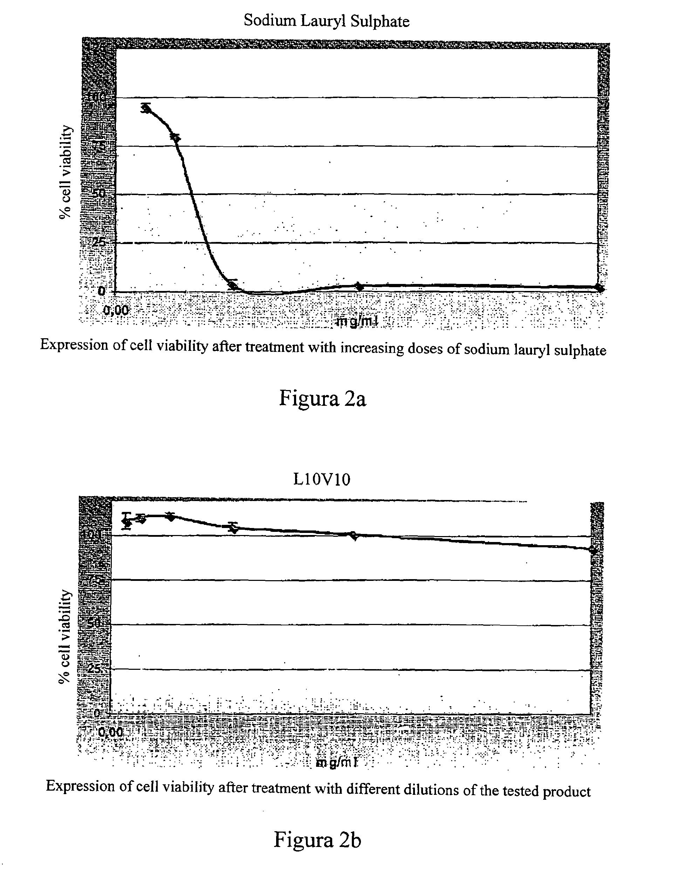

The cytotoxicity data obtained with the MTT test, corrected subtracting the absorbance readings due to the diluent medium, were plotted against the concentration of the tested product, thus generating a dose-response curve, shown in Figure 2a for SLS and 2b for L10V10, which allowed to determine the theoretical regression curve for evaluating the concentration of 50% inhibition of cell growth (IC$_{50}$).

The IC$_{50}$ value for SLS was 0.07 mg/ml, while that one for L10V10 was IC$_{50}$> 5 mg/ml.

On the basis of the above mentioned results, the product anti-wrinkle cream for sensitive skins L10V10 did not show any cytotoxic effect on human keratinocytes. These data are thus deemed predictive of absence of irritating effects in vivo.

**Example 8**

In vitro analysis of the pro-sensitising potential of the Anti-wrinkle cream for sensitive skins L10V10

The evaluation of pro-sensitising effects by the cream L10V10 was carried out as in example 6, but using anti-wrinkle cream for sensitive skins L10V10 instead of cream L9V9.

The test was thus performed again on a monocyte cellular line named THP-1, and the modulation in the expression of the two co-stimulatory molecules, CD80 (B7.1) and CD86 (B7.2) was then evaluated, by using as positive control Nickel Sulphate (NiSO46H2O) in crystals dissolved in PBS and after a preliminary evaluation in order to obtain the use concentration of cream and of nickel sulphate.

Morphological qualitative parameters (cell volume change, modifications in the cellular granulations) linked to cellular necrosis and apoptosis were detected again, and as control (basal fluorescence) MFI of both untreated THP-1 cells (apart from washings in PBS) and THP-1 cells reacted with a fluresceinated monoclonal antibody (as those anti-B7.1 and anti-B7.2) but of irrelevant specificity (isotype-matched control) was evaluated again.

Table 4 below shows the results of the analysis of the monocyte cell line THP-1 for expression of co-stimulatory molecules at flux cytofluorimeter after 48 hours of reaction with the samples at the two tested concentrations and with the controls, corrected by the negative control.

Table 4

| Samples | CD80 (MFI*) | CD86 (MFI*) |
|---|---|---|
| Nickel sulphate 20 $\mu$g/ml | 29.00 | 77.12 |
| Nickel sulphate 10 $\mu$g/ml | 19.85 | 37.35 |
| Nickel sulphate 4 $\mu$g/ml | 0.41 | 0.79 |
| anti-wrinkle cream for sensitive skins L10V 10 10mg/ml | -0.06 | -1.12 |
| anti-wrinkle cream for sensitive skins L10V10 2 mg/ml | -0.07 | -1.01 |
| *MFI = Mean Fluorescente Intensity, i.e. the geometric average of the fluorescence intensity of the cells stained with the fluoresceinated antibody and it is proportional to the number of stained molecules per cell. | | |

By observing the behaviour of Nickel, a typical allergising substance, it was seen how this substance was characterised by a) a high increase of both markers; b) direct correlation between the increase of intensity of the response and the concentration; c) detactable effect even at very low doses.

The tested dose of 4 $\mu$g/ml of Nickel Sulphate ($NiSO_4 6H_2O$) corresponded to about 1 ppm of Nickel, a value that was around the allergising threshold in already sensitised individuals and on irritated skin (Gawkrodger DJ, Nickel dermatitis: how much Nickel is safe? Contact Dermatitis 1996; 35:267-271; Tanojo H, Hostynek JJ, Mountford HB, Mibach HI, In vitro premeation of nickel salts through human stratum corneum. Acta Derm Venereol Suppl, 2001; 212: 19-23). During the test it was verified how this concentration was able of causing a detectable increase in CD80, with respect to untreated controls (data not shown).

The concentration able to cause an allergic reaction in most of the sensitive subjects was anyway around much higher values, above 100 ppm of Nickel Sulphate $6H_2O$, in contact with healthy and intact skin.

At the tested concentrations, cream L10V10 had not shown any modulation of the investigated markers.

Cream L10V10 had not shown any cytotoxic effects on the cells used for the test ($IC_{50}$= 50 mg/ml). At the used dilutions, no apoptic effects on the examined cells came out.

In the above test the finished cosmetic product anti-wrinkle cream for sensitive skins L10V10 didn't increase the expression of any investigated marker in human monocytes in this *in vitro* model, thus showing it does not have any stimulatory potential on the immune system mediated by monocyte/macrophage.

**Example 9**

Cytotoxicity evaluation of moisturizing cream L11V11 with MTT method

The cytotoxicity evaluation test of the moisturizing cream for sensitive skins of example 5 was repeated, but using samples of moisturizing cream for sensitive skins L 11 V11 as prepared in example 3.

Specifically, the same cell type (stabilized human keratinocytes (HaCaT cells)), the same equipment for the evaluation, the same culture medium, and the same amounts of cream were employed, and the same method was performed as in example 5. Untreated cells were used as negative control, while as positive control cells were treated with Sodium Lauryl Sulphate (SLS), dissolved in the culture medium at the same concentrations as in example 5 (0.5, 0.25, 0.12, 0.06 and 0.03 mg/ml).

At the end of the incubation period of each well, the MTT-culture medium was removed and a MTT solubilizing solution was added to each well as in example 5.

The plate was then stirred on a plate stirrer, ensuring that all the crystals were dissolved and had formed a homogeneous solution.

The absorbance was then read with a chromatometer equipped with a plate reader by subtracting background reading, thus obtaining the percentage of cell survival.

The results obtained for SLS and L 11 V 11 were as set forth in Table 5 below.

**Table 5: cell viability (%) for SLS and cream L11V11**

| SLS (positive control) | | | | | |
|---|---|---|---|---|---|
| Dose mg/ml | 0.5 | 0.25 | 0.12 | 0.06 | 0.03 |
| Cell viability % (vs negative control) | 2.3 | 2.3 | 2.5 | 61.0 | 87.6 |
| stand dev. | 0.9 | 0.7 | 1.1 | 4.1 | 4.6 |
| Moisturizing cream for sensitive skins L11V11 | | | | | |
| Dose mg/ml | 5 | 2.5 | 1.25 | 0.6 | 0.3 | 0.15 |
| Cell viability % (vs negative control) | 102.4 | 109.6 | 104.7 | 106.3 | 102.6 | 96.2 |
| Stand. dev. | 2.9 | 6.5 | 6.8 | 10.3 | 9.4 | 8.8 |

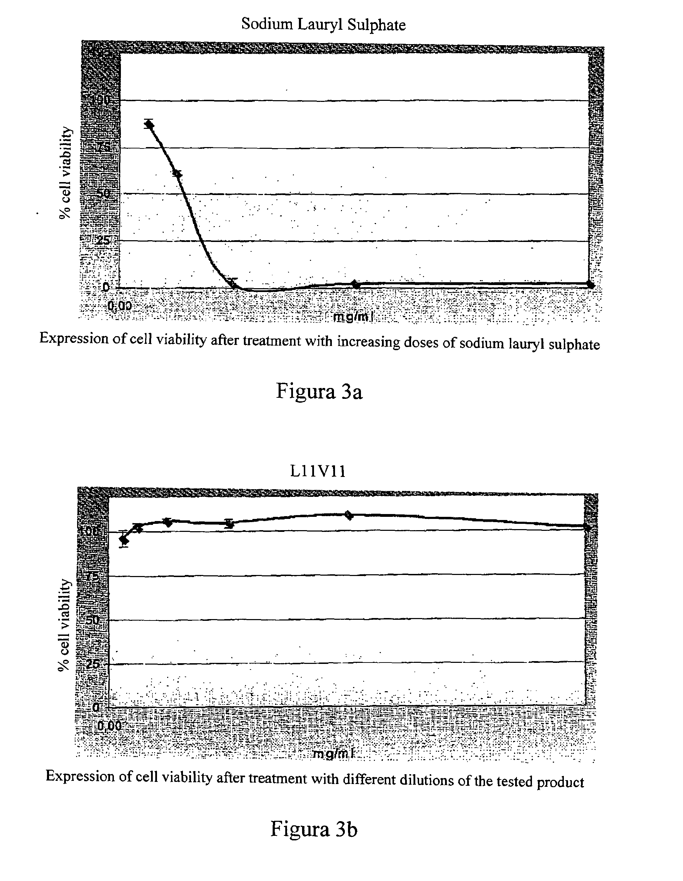

The cytotoxicity data obtained with the MTT test, corrected subtracting the absorbance readings due to the diluent medium, were plotted against the concentration of the tested product, thus generating a dose-response curve, shown in Figure 3a for SLS and 3b for L11V11, which allowed to determine the theoretical regression curve for evaluating the concentration of 50% inhibition of cell growth ($IC_{50}$).

The $IC_{50}$ value for SLS was 0.06 mg/ml, while that one for L11V11 was $IC_{50} > 5$ mg/ml.

On the basis of the above mentioned results, the product Moisturizing cream for sensitive skins L 11 V 11 did not show any cytotoxic effects on human keratinocytes. These data are thus deemed predictive of absence of irritating effects in vivo.

**Example 10**

In vitro analysis of the pro-sensitising potential of moisturizing cream for sensitive skins L11V11

The evaluation of pro-sensitising effects by the cream L11V11 was carried out as in example 6, but using the moisturizing cream for sensitive skins L11 V 11 instead of cream L9V9.

The test was thus performed again on a monocyte cellular line named THP-1, and the expression modulation of the two co-stimulatory molecules, CD80 (B7.1) and CD86 (B7.2) was then evaluated, by using as positive control Nickel Sulphate ($NiSO_4 6H_2O$) in crystals dissolved in PBS and after a preliminary evaluation in order to obtain the use concentration of cream and of nickel sulphate.

Morphological qualitative parameters (cell volume change, modifications in the cellular granulations) linked to cellular necrosis and apoptosis were detected again, and as control (basal fluorescence) MFI of both untreated THP-1 cells (apart from washings in PBS) and THP-1 cells reacted with a fluresceinated monoclonal antibody (as the anti-B7.1 and anti-B7.2) but of irrelevant specificity (isotype-matched control) was evaluated again.

Table 6 below shows the results of the analysis of monocyte cell line THP-1 for expression of co-stimulatory molecules at flux cytofluorimeter after 48 hours of reaction with the samples at the two tested concentrations and with the controls, corrected by the negative control.

**Table 6**

| Samples | CD80 (MFI*) | CD86 (MFI*) |
|---|---|---|
| Nickel sulphate 20 μg/ml | 29.00 | 77.12 |

(continued)

| Samples | CD80 (MFI*) | CD86 (MFI*) |
|---|---|---|
| Nickel sulphate 10 $\mu$g/ml | 19.85 | 37.35 |
| Nickel sulphate 4 $\mu$g/ml | 0.41 | 0.79 |
| moisturizing cream for sensitive skins L 11 V11 5mg/ml | -0.03 | -1.01 |
| moisturizing cream for sensitive skins L 11 V11 1 mg/ml | -0.07 | -1.11 |
| *MFI = Mean Fluorescente Intensity, i.e. the geometric average of the fluorescence intensity of the cells stained with the fluoresceinated antibody and it is proportional to the number of stained molecules per cell. | | |

By observing the behaviour of Nickel, a typical allergising substance, it was seen how this substance was characterised by a) a high increase of both markers; b) direct correlation between the increase of intensity of the response and the concentration; c) detactable effect even at very low doses.

The tested dose of 4 $\mu$g/ml of Nickel Sulphate ($NiSO_46H_2O$) corresponded to about 1 ppm of Nickel, a value that was around the allergising threshold in already sensitised individuals and on irritated skin (Gawkrodger DJ, Nickel dermatitis: how much Nickel is safe? Contact Dermatitis 1996; 35:267-271; Tanojo H, Hostynek JJ, Mountford HB, Mibach HI, In vitro premeation of nickel salts through human stratum corneum. Acta Derm Venereol Suppl, 2001; 212: 19-23). During the test it was verified how this concentration was able of causing a detectable increase in CD80, with respect to untreated controls (data not shown).

The concentration able to cause an allergic reaction in most of the sensitive subjects was anyway around much higher values, above 100 ppm of Nickel Sulphate $6H_2O$, in contact with healthy and intact skin.

At the tested concentrations, cream L11V11 had not shown any modulation of the investigated markers.

The cream L11V11 had not shown any cytotoxic effect on the cells used for the test ($IC_{50}$= 25 mg/ml). At the used dilutions, no apoptotic effects on the examined cells came out.

In the above test the finished cosmetic product Moisturizing cream for sensitive skins L 11 V11 didn't increase the expression of any investigated marker in human monocytes in this *in vitro* model, thus showing that it does not have any stimulatory potential on the immune system mediated by monocyte/macrophage.


**Example 11**

Cytotoxicity evaluation of anti-wrinkle cream L12V 12 with MTT method

The cytotoxicity evaluation test of the moisturizing cream for sensitive skins of example 5 was repeated, but using samples of anti-wrinkle cream for sensitive skins L12V12 as prepared in example 4.

Specifically, the same cell type (stabilized human keratinocytes (HaCaT cells)), the same equipment for the evaluation, the same culture medium, and the same amounts of cream were employed, and the same method was performed as in example 5. Untreated cells were used as negative control, while as positive control cells were treated with Sodium Lauryl Sulphate (SLS), dissolved in the culture medium at the same concentrations as in example 5 (0.5, 0.25, 0.12, 0.06 and 0.03 mg/ml).

At the end of the incubation period of each well, MTT-culture medium was removed and a MTT solubilizing solution was added to each well as in example 5.

The plate was then stirred on a plate stirrer, ensuring that all the crystals were dissolved and had formed a homogeneous solution.

The absorbance was then read with a chromatometer equipped with a plate reader by subtracting background reading, thus obtaining the percentage of cell survival.

The results obtained for SLS and L12V12 were as set forth in Table 7 below.

**Table 7: cell viability (%) for SLS and cream L12V12**

| SLS (positive control) | | | | | |
|---|---|---|---|---|---|
| Dose mg/ml | 0.5 | 0.25 | 0.12 | 0.06 | 0.03 |
| Cell viability % (vs negative control) | 2.2 | 2.9 | 3.9 | 79.6 | 94.7 |
| Stand. dev. | 0.8 | 0.6 | 0.6 | 8.4 | 7.7 |

(continued)

| Anti-wrinkle cream for sensitive skins L12V12 | | | | | | |
|---|---|---|---|---|---|---|
| Dose mg/ml | 5 | 2.5 | 1.25 | 0.6 | 0.3 | 0.15 |
| Cell viability % (vs negative control) | 101.7 | 107.4 | 109.7 | 103.2 | 96.8 | 97.8 |
| Stand. dev. | 2.6 | 4.7 | 6.1 | 3.7 | 2.3 | 4.6 |

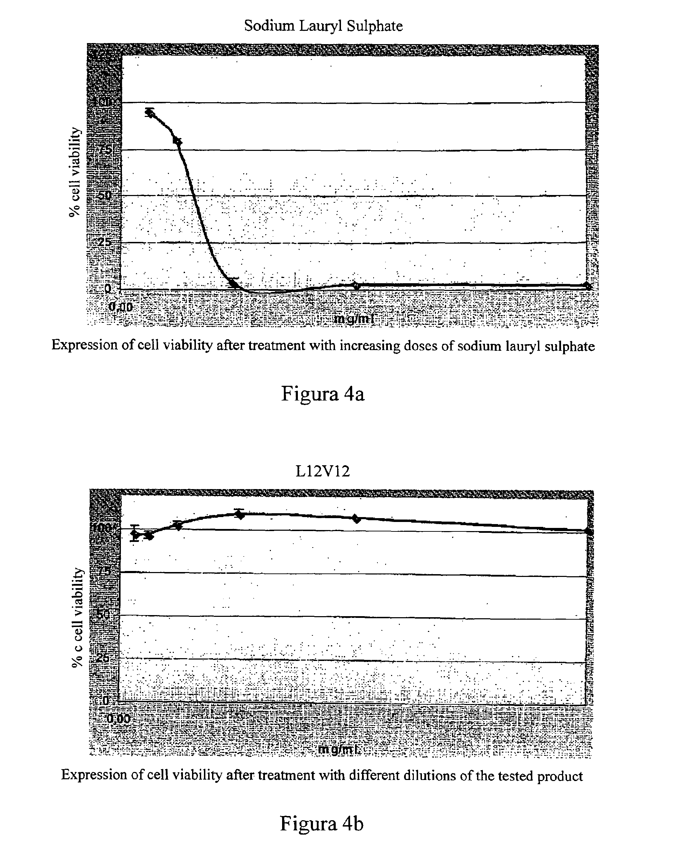

The cytotoxicity data obtained with the MTT test, corrected by subtracting the absorbance readings due to the diluent medium, were plotted against the concentration of the tested product, thus generating a dose-response curve, shown in Figure 4a for SLS and 4b for L12V12, which allowed to determine the theoretical regression curve for evaluating the concentration of 50% inhibition of cell growth ($IC_{50}$).

The $IC_{50}$ value for SLS was 0.07 mg/ml, while that one for L12V12 was $IC_{50}$> 5 mg/ml.

On the basis of the above mentioned results, the product anti-wrinkle cream for sensitive skins L12V 12 did not show any cytotoxic effect on human keratinocytes. These data are thus deemed predictive of absence of irritating effects in vivo.

**Example 12**

In vitro analysis of the pro-sensitising potential of anti-wrinkle cream for sensitive skins L12V 12

The evaluation of pro-sensitising effects by the cream L12V12 was carried out as in example 6, but using anti-wrinkle cream for sensitive skins L12V 12 instead of cream L9V9.

The test was thus performed again on a monocyte cellular line named THP-1, and the expression modulation of the two co-stimulatory molecules, CD80 (B7.1) and CD86 (B7.2) was then evaluated, by using as positive control Nickel Sulphate ($NiSO_4 6H_2O$) in crystals dissolved in PBS and after a preliminary evaluation in order to obtain the use concentration of cream and of nickel sulphate.

Morphological qualitative parameters (cell volume change, modifications in the cellular granulations) linked to cellular necrosis and apoptosis were detected again, and as control (basal fluorescence) MFI of both untreated THP-1 cells (apart from washings in PBS) and THP-1 cells reacted with a fluresceinated monoclonal antibody (as those anti-B7.1 and anti-B7.2) but of irrelevant specificity (isotype-matched control) was evaluated again.

Table 8 below shows the results of the analysis of the monocyte cell line THP-1 for expression of co-stimulatory molecules at flux cytofluorimeter after 48 hours of reaction with the samples at the two tested concentrations and with the controls, corrected by the negative control.

**Table 8**

| Samples | CD80 (MFI*) | CD86 (MFI*) |
|---|---|---|
| Nickel sulphate 20 μg/ml | 29.00 | 77.12 |
| Nickel sulphate 10 μg/ml | 19.85 | 37.35 |
| Nickel sulphate 4 μg/ml | 0.41 | 0.79 |
| anti-wrinkle cream for sensitive skins L12V12 10 mg/ml | -0.26 | -0.88 |
| anti-wrinkle cream for sensitive skins L12V 12 2 mg/ml | -0.28 | -0.64 |
| *MFI = Mean Fluorescente Intensity, i.e. the geometric average of the fluorescence intensity of the cells stained with the fluresceinated antibody and it is proportional to the number of stained molecules per cell. | | |

By observing the behaviour of Nickel, a typical allergising substance, it was seen how this substance was characterised by a) a high increase of both markers; b) direct correlation between the increase of intensity of the response and the concentration; c) detactable effect even at very low doses.

The tested dose of 4 μg/ml of Nickel Sulphate ($NiSO_4 6H_2O$) corresponded to about 1 ppm of Nickel, a value that was around the allergising threshold in already sensitised individuals and on irritated skin (Gawkrodger DJ, Nickel dermatitis: how much Nickel is safe? Contact Dermatitis 1996; 35:267-271; Tanojo H, Hostynek JJ, Mountford HB, Mibach HI, In vitro premeation of nickel salts through human stratum corneum. Acta Derm Venereol Suppl, 2001; 212:

19-23). During the test it was verified how this concentration was able of causing a detectable increase in CD80, with respect to untreated controls (data not shown).

The concentration able to cause an allergic reaction in most of the sensitive subjects was anyway around much higher values, above 100 ppm of Nickel Sulphate $6H_2O$, in contact with healthy and intact skin.

At the tested concentrations, anti-wrinkle cream L12V12 had not shown any modulation of the investigated markers.

Anti-wrinkle cream L12V12 had not shown any cytotoxic effect on the cells used for the test ($IC_{50}$= 50 mg/ml). At the used dilutions, no apoptotic effects on the examined cells came out.

In the above test the finished cosmetic product Anti-wrinkle cream for sensitive skins L12V12 didn't increase the expression of any investigated marker in human monocytes in this *in vitro* model, thus showing it does not have any stimulatory potential on the immune system mediated by monocyte/macrophage.

**Comparative example 13**

The tests of cytotoxicity and sensitising potential evaluation were repeated on two commercial products known for the treatment of sensitive skins, but containing a lower amount of preservatives.

Specifically, the tested creams of prior art were:

1) cream for delicate skins containing as preservatives triethanolamine, methylparaben, ethylparaben, propylparaben, butylparaben, sodium dehydroacetate, and named as sample A.

2) anti-wrinkle cream for sensitive skins containing as preservatives triethanolamine, propylparaben, butylparaben, phenoxyethanol, and named as sample B.

Cytotoxicity evaluation

The cytotoxicity evaluation test of the moisturizing cream for sensitive skins of example 5 was repeated, but using firstly sample A and then sample B of prior art.

Specifically, the same cell type (stabilized human keratinocytes (HaCaT cells)), the same equipment for the evaluation, the same culture medium, and the same amounts of cream were employed, and the same method was performed as in example 5. Untreated cells were used as negative control, while as positive control cells were treated with Sodium Lauryl Sulphate (SLS), dissolved in the culture medium at the same concentrations as in example 5 (0.5, 0.25, 0.12, 0.06 and 0.03 mg/ml).

At the end of the incubation period of each well, MTT-culture medium was removed and a MTT solubilizing solution was added to each well as in example 5.

The plate was then stirred on a plate stirrer, ensuring that all the crystals were dissolved and had formed a homogeneous solution.

The absorbance was then read with a chromatometer equipped with a plate reader by subtracting background reading, thus obtaining the percentage of cell survival.

The results obtained for SLS and sample A were as set forth in the table 9 and those for sample B in table 10 below:

**Table 9: cell viability results for SLS and sample A**

| SLS (positive control) | | | | | |
|---|---|---|---|---|---|
| Dose mg/ml | 0.5 | 0.25 | 0.12 | 0.06 | 0.03 |
| Cell viability % (vs negative control) | 0.2 | 0.1 | 0.2 | 76.7 | 98.5 |
| Stand. dev. | 1.2 | 1.1 | 1.2 | 8.2 | 6.4 |
| Sample A | | | | | |
| Dose mg/ml | 5 | 2.5 | 1.25 | 0.6 | 0.3 | 0.15 |
| Cell viability % (vs negative control) | 40.2 | 45.8 | 53.7 | 74.8 | 81.4 | 85.4 |
| Stand. dev. | 1.3 | 2.7 | 6.3 | 3.3 | 2.7 | 7.7 |

**Table 10: cell viability results for SLS and sample B**

| SLS (positive control) | | | | | | |
|---|---|---|---|---|---|---|
| Dose mg/ml | | 0.5 | 0.25 | 0.12 | 0.06 | 0.03 |
| Cell viability % (vs negative control) | | 1.2 | 1.5 | 1.9 | 73.9 | 92.7 |
| Stand. dev. | | 1.1 | 1.4 | 1.0 | 0.7 | 4.0 |
| Sample B | | | | | | |
| Dose mg/ml | 5 | 2.5 | 1.25 | 0.6 | 0.3 | 0.15 |
| Cell viability % (vs negative control) | 75.5 | 76.3 | 75.7 | 76.9 | 78.9 | 82.4 |
| stand dev. | 1.5 | 1.4 | 3.7 | 3.3 | 5.0 | 3.9 |

The cytotoxicity data obtained with MTT test, corrected by subtracting the absorbance readings due to the diluent medium, were plotted against the concentration of the tested product, thus generating a dose-response curve, which allowed to determine the theoretical regression curve for evaluating the concentration of 50% inhibition of cell growth ($IC_{50}$).

The $IC_{50}$ value for SLS was 0.07 mg/ml as compared to sample A and 0.067 mg/ml as compared to sample B.

Sample A showed $IC_{50}$= 1.85 mg/ml.
Sample B showed $IC_{50}$> 5 mg/ml.

On the basis of the above mentioned results, sample A product, although deemed non-irritating and having discrete biocompatibility values, was lower than sample B, which instead showed very high biocompatibility and had not shown any cytotoxic effect on human keratinocytes.

The matrix according to the invention and the cosmetic formulation containing it of examples 5, 7, 9 and 11 showed biocompatibility values similar to sample B, although comprising no preservative in the formulation.

Evaluation of pro-sensitising effects

The evaluation of sample A and sample B was carried out as in example 6, but using firstly sample A and then sample B instead of cream L9V9.

The tests were then performed again on a monocyte cellular line named THP-1, and the modulation in the expression of the two co-stimulatory molecules, CD80 (B7.1) and CD86 (B7.2) was then evaluated, using as positive control Nickel Sulphate ($NiSO_4 6H_2O$) in crystals dissolved in PBS and after a preliminary evaluation in order to obtain the use concentration of the comparative creams and of nickel sulphate.

Morphological qualitative parameters (cell volume change, modifications in the cellular granulations) linked to cellular necrosis and apoptosis were detected again, and as control (basal fluorescence) MFI of both untreated THP-1 cells (apart from washings in PBS) and THP-1 cells reacted with a fluresceinated monoclonal antibody (as those anti-B7.1 and anti-B7.2) but of irrelevant specificity (isotype-matched control) was evaluated again.

Table 11 below shows the results of the analysis of the monocyte cell line THP-1 for expression of co-stimulatory molecules at flux cytofluorimeter after 48 hours of reaction with the samples at the two tested concentrations of sample A and with the controls, corrected by the negative control.

**Table 11: results sample A and nickel sulphate**

| Samples | CD80 (MFI*) | CD86 (MFI*) |
|---|---|---|
| Nickel sulphate 20 $\mu$g/ml | 33.48 | 87.92 |
| Nickel sulphate 10 $\mu$g/ml | 18.33 | 33.26 |
| Nickel sulphate 4 $\mu$g/ml | 0.02 | -4.54 |
| cream sample A 4 mg/ml | -0.02 | 0.00 |

(continued)

| Samples | CD80 (MFI*) | CD86 (MFI*) |
|---|---|---|
| cream sample A 0.8 mg/ml | -0.19 | -0.10 |
| *MFI = Mean Fluorescente Intensity, i.e. the geometric average of the fluorescence intensity of the cells stained with the fluoresceinated antibody and it is proportional to the number of stained molecules per cell. | | |

Table 12 below shows the results of the analysis of the monocyte cell line THP-1 for expression of co-stimulatory molecules at flux cytofluorimeter after 48 hours of reaction with the samples at the two tested concentrations of sample B and with the controls, corrected by the negative control.

**Table 12: results sample B and nickel sulphate**

| Samples | CD80 (MFI*) | CD86 (MFI*) |
|---|---|---|
| Nickel sulphate 20 $\mu$g/ml | 29.00 | 77.12 |
| Nickel sulphate 10 $\mu$g/ml | 19.85 | 37.35 |
| Nickel sulphate 4 $\mu$g/ml | 0.41 | 0.79 |
| cream sample B 5mg/ml | 0 | -0.54 |
| cream sample B 1 mg/ml | -0.03 | -0.34 |
| *MFI = Mean Fluorescente Intensity, i.e. the geometric average of the fluorescence intensity of the cells stained with the fluoresceinated antibody and it is proportional to the number of stained molecules per cell. | | |

At the tested concentrations, the creams of both sample A and B had not shown any modulation of the investigated markers.

Sample A had not shown any cytotoxic effect on the cells used for the test ($IC_{50}$= 20 mg/ml). At the used dilutions, no apoptotic effects on the examined cells came out. Sample B had not shown any cytotoxic effect on the cells used for the test ($IC_{50}$= 25 mg/ml). At the used dilutions, no apoptotic effects on the examined cells came out.

The matrix according to the invention and the cosmetic formulation containing it of examples 6, 8, 10 and 12 showed it does not increase the expression of any investigated marker in human monocytes in this in vitro model, thus showing it does not have any stimulatory potential on the immune system mediated by monocyte/macrophageha, at levels even higher than the comparative creams of samples A and B.

**Example 14**

Test to evaluate the preserving system efficacy in finished cosmetic products for the Moisturizing cream for sensitive skins L9V9

The Challenge test is a predictive method aimed at evaluating the efficacy of a preserving system employed in the formulation of a cosmetic product, reproducing in laboratory the pollution conditions by micro-organisms to which cosmetic products can be subjected during processing, storage and normal use by the consumer.

As the preparation of the product doesn't occur in sterility conditions, there is always a certain level of basic microbial contamination which must be controlled by a suitable preserving system. Furthermore, the use of the product by the consumer implies further contaminations which reoccur throughout the all time of use.

When performing the test in laboratory, there is a tendency to intentionally exaggerate the experimental conditions inoculating the product with a very high concentration of micro-organisms, which are hardly likely to occur in real life.

A mixed inoculum of five different micro-organisms strains was performed twice in the product L9V9, as reported below, and the percentage of decrease in the microbial charge was evaluated at different times.

The microbiological evaluation was performed before the inoculum and it was found that, through plate count, the total mesophilic bacteria charge and yeasts and moulds were <10 U.F.C./g or ml.

The inoculum was performed with five microbial strains at different concentrations, as reported in Table 13 below:

**Table 13:**

| STRAIN | Inoculum concentration in the sample (UFC/g) |
|---|---|
| *Escherichia coli ATCC 8739* | $1.5 \times 10^6$ |
| *Pseudomonas aeruginosa ATCC 9027* | $3.1 \times 10^6$ |
| *Staphylococcus aureus ATCC 6538* | $6.5 \times 10^6$ |
| *Candida albicans ATCC 10231* | $4.4 \times 10^4$ |
| *Aspergillus niger ATCC 16404* | $1.4 \times 10^5$ |

The inoculum was performed separately for mixed yeasts and moulds, mixed Gram- bacteria and Gram+ bacteria. The concentration of viable cells was determined via the plate count method.

The treated packages ware then maintained at room temperature until the subsequent cultures were made. The count of micro-organisms at different times was performed collecting 1 g of product and diluting it up to $1 \times 10^6$ times. Each dilution was then plated in Petri plates containing the agarized selective culture medium. The plates were incubated in a thermostat at 35°C (bacteria) or at 22°C (yeasts and moulds) for the time necessary to allow a micro-organism growth suitable for performing the count (3 days for bacteria, 5-7 days for yeasts and moulds).

By correcting the number of observed colonies by the dilution factor, the U.F.C. (Colony-forming units) value was obtained per gram of product. Together with the zero time culture, three cultures were performed after 24 hours, 7, 14 and 28 days in order to evaluate the dynamics of the microbial removal.

The microbiological stability was deemed adequate when at the Challenge the microbic growth was reduced by > 99.9% for bacteria and by > 90% for yeasts and moulds after seven days from the inoculum, without subsequent regrowth.

The results obtained are reported in the following table:

Total microbial count:

| Time | *Escherichia coli UFC/q* | *Pseudomonas aeruginosa UFC/g* | *Staphylococcus aureus UFC/g* | *Candida albicans UFC/g* | *Aspergillus niger UFC/g* |
|---|---|---|---|---|---|
| 24 hours | <10 | < 10 | $1.8 \times 10^5$ | < 10 | $1.7 \times 10^4$ |
| 7 days | <10 | <10 | <10 | < 10 | $6.0 \times 10^2$ |
| 14 days | <10 | <10 | <10 | <10 | <10 |
| 28 days | <10 | <10 | <10 | <10 | <10 |

Reduction in microbial growth:

| Time | *Escherichia coli UFC/g* | *Pseudomonas aeruginosa UFC/g* | *Staphylococcus aureus UFC/g* | *Candida albicans UFC/g* | *Aspergillus niger UFC/g* |
|---|---|---|---|---|---|
| after 7 days | > 99.9% | > 99.9% | > 99.9% | > 99.9% | > 90.0% |
| after 28 days | > 99.9% | > 99.9% | > 99.9% | > 99.9% | > 99.9% |

For Escherichia coli, Pseudomonas aeruginosa, Staphylococcus aureus and Candida Albicans strains the microbial charge reduction turned out to be above 99.9% after 7 days and after 28 days. For Aspergillus niger strain the microbial charge reduction turned out to be above 90% after 7 days and above 99.9% after 28 days. The preserving system of moisturizing cream for sensitive skins L9V9 was thus deemed effective towards all tested strains.

### Example 15

Test to evaluate the preserving system efficacy in finished cosmetic products for anti-wrinkle cream for sensitive skins L10V10

An evaluation example of the preserving system was repeated as in example 14, but using a sample of anti-wrinkle cream for sensitive skins L10V 10 as prepared in example 2.

Before the inoculum, the cream was evaluated and it was seen that the charge, the total mesophilic bacteria charge and yeasts and moulds, through plate count, were <10 U.F.C./g or ml.

The inoculum was performed with the same five bacteria strains as in example 14 at the same concentrations (see Table 13), and the followed method was the same as above.

The following results were obtained:

Total microbial count:

| Time | Escherichia coli UFC/g | Pseudomonas aeruginosa UFC/g | Staphylococcus aureus UFC/g | Candida aibicans UFC/g | Aspergillus niger UFC/g |
|---|---|---|---|---|---|
| 24 hours | < 10 | <10 | $4.3 \times 10^4$ | < 10 | $1.1 \times 10^4$ |
| 7 days | <10 | <10 | < 10 | < 10 | $6.6 \times 10^2$ |
| 14 days | <10 | <10 | <10 | < 10 | <10 |
| 28 days | <10 | < 10 | <10 | < 10 | <10 |

Reduction in microbial growth:

| Time | Escherichia coli UFC/g | Pseudomonas aeruginosa UFC/g | Staphylococcus aureus UFC/g | Candida aibicans UFC/g | Aspergillus niger UFC/g |
|---|---|---|---|---|---|
| after 7 days | > 99.9% | > 99.9% | > 99.9% | > 99.9% | > 90.0% |
| after 28 days | > 99.9% | > 99.9% | > 99.9% | > 99.9% | > 99.9% |

For Escherichia coli, Pseudomonas aeruginosa, Staphylococcus aureus and Candida Albicans strains the microbial charge reduction turned out to be above 99.9% after 7 days and after 28 days. For Aspergillus niger strain the microbial charge reduction turned out to be above 90% after 7 days and above 99.9% after 28 days. The preserving system of anti-wrinkle cream for sensitive skins L10V10 was thus deemed effective towards all tested strains.

### Example 16

Test to evaluate the preserving system efficacy in finished cosmetic products for moisturizing cream for sensitive skins L11V11

An evaluation example of the preserving system was repeated as in example 14, but using a sample of Moisturizing cream for sensitive skins L11V11 as prepared in example 3.

Before the inoculum, the cream was evaluated and it was seen that the charge, the total mesophilic bacteria charge and yeasts and moulds, through plate count, were <10 U.F.C./g or ml.

The inoculum was performed with the same five bacteria strains as in example 14 at the same concentrations (see Table 13), and the followed method was the same as above.

The following results were obtained:

Total microbial count:

| Time | Escherichia coli UFC/q | Pseudomonas aeruginosa UFC/g | Staphylococcus aure us UFC/g | Candida albicans UFC/g | Aspergillus niger UFC/g |
|---|---|---|---|---|---|
| 24 hours | < 10 | < 10 | $4.4 \times 10^4$ | <10 | $1.0 \times 10^4$ |
| 7 days | <10 | <10 | <10 | <10 | $2.9 \times 10^2$ |
| 14 days | <10 | <10 | <10 | <10 | <10 |
| 28 days | <10 | <10 | <10 | <10 | <10 |

Reduction in microbial growth:

| Time | Escherichia coli UFC/g | Pseudomonas aeruginosa UFC/g | Staphylococcus aure us UFC/g | Candida albicans UFC/g | Aspergillus niger UFC/g |
|---|---|---|---|---|---|
| after 7 days | > 99.9% | > 99.9% | > 99.9% | > 99.9% | > 90.0% |
| after 28 days | > 99.9% | > 99.9% | > 99.9% | > 99.9% | > 99.9% |

For Escherichia coli, Pseudomonas aeruginosa, Staphylococcus aureus and Candida Albicans strains the microbial charge reduction turned out to be above 99.9% after 7 days and after 28 days. For Aspergillus niger strain the microbial charge reduction turned out to be above 90% after 7 days and above 99.9% after 28 days. The preserving system of moisturizing cream for sensitive skins L11 V11 was thus deemed effective towards all tested strains.

**Example 17**

<u>Test to evaluate the preserving system efficacy in finished cosmetic products for anti-wrinkle cream for sensitive skins L12V12</u>

An evaluation example of the preserving system was repeated as in example 14, but using a sample of Anti-wrinkle cream for sensitive skins L12V12 as prepared in example 4.
Before the inoculum, the cream was evaluated and it was seen that the charge, the total mesophilic bacteria charge and yeasts and moulds, through plate count, were <10 U.F.C./g or ml.
The inoculum was performed with the same five bacteria strains as in example 14 at the same concentrations (see Table 13), and the followed method was the same as above.
The following results were obtained:
Total microbial count:

| Time | Escherichia coli UFC/g | Pseudomonas aeruginosa UFC/g | Staphylococcus aureus UFC/g | Candida albicans UFC/g | Aspergillus niger UFC/g |
|---|---|---|---|---|---|
| 24 hours | <10 | < 10 | $3.0 \times 10^4$ | < 10 | $6.0 \times 10^3$ |
| 7 days | <10 | <10 | <10 | <10 | 70 |
| 14 days | <10 | <10 | <10 | <10 | <10 |
| 28 days | <10 | <10 | <10 | <10 | <10 |

Reduction in microbial growth:

| Time | Escherichia coli UFC/g | Pseudomonas aeruginosa VFC7g | Staphylococcus aureus UFC/g | Candida albicans UFC/g | Aspergillus niger UFC/g |
|---|---|---|---|---|---|
| after 7 days | > 99.9% | > 99.9% | > 99.9% | > 99.9% | > 90.0% |

(continued)

| Time | Escherichia coli UFC/g | Pseudomonas aeruginosa VFC7g | Staphylococcus aureus UFC/g | Candida albicans UFC/g | Aspergillus niger UFC/g |
|---|---|---|---|---|---|
| after 28 days | > 99.9% | > 99.9% | > 99.9% | > 99.9% | > 99.9% |

For Escherichia coli, Pseudomonas aeruginosa, Staphylococcus aureus and Candida Albicans strains the microbial charge reduction turned out to be above 99.9% after 7 days and after 28 days. For Aspergillus niger strain the microbial charge reduction turned out to be above 90% after 7 days and above 99.9% after 28 days. The preserving system of anti-wrinkle cream for sensitive skins L12V12 was thus deemed effective towards all tested strains.

Therefore, the above evaluation examples showed that the matrix of the invention allows to prepare a desired, preservative-free, cosmetic formulation, extremely safe and suitable for sensitive skins.

The invention has been described with reference to some examples of cream production, but it will be possible to make changes, such as adding appropriate excipients to prepare a gel, a lipogel, or a lotion, without departing from the scope of the appended claims.

## Claims

1. A water-based matrix for preparing preservative-free cosmetic preparations comprising:

   - 2 to 40% by weight of at least one vegetable ester of glycerin with one or more mono- or polyunsaturated fatty acids, having 18 to 20 carbon atoms;
   - 0.5 to 20% by weight of at least one non-triglyceride ester formed from an alcohol having 12 to 22 carbon atoms, and one or more saturated, monounsaturated and/or polyunsaturated fatty acids having 14 to 22 carbon atoms;
   - 0.5 to 25% by weight of at least one polyol of functionality 2 to 12 and pentylene glycol;
   - at least 0.012% by weight of at least one sequestrating agent;
   - 0.2% to 20% by weight of at least one antioxidant agent;
   - 0.0001% to 5% by weight of at least one unsaponifiable fraction of a natural oil and/or natural butter containing sterolic fractions of the natural plant from which it derives.

2. The water-based matrix according to claim 1, wherein the at least one vegetable ester of glycerin with one or more mono- and/or polyunsaturated fatty acids, having 18 to 20 carbon atoms is selected from the group consisting of sweet almond oil, wheat germ oil, olive oil, avocado oil, argan oil, soybean oil, rapeseed oil, corn oil, rice oil, borage oil, macadamia oil, evening primrose oil (oenothera oil), rose hip seed oil (rose mosqueta oil), soybean glycerides, shea butter (karite butter), crab oil, hazelnut butter, illipe butter and mixtures thereof.

3. The water-based matrix according to claim 2, wherein the at least one vegetable ester of glycerin with one or more mono- and/or polyunsaturated fatty acids, having 18 to 20 carbon atoms is selected from the group consisting of soybean oil, wheat seed oil, soybean glycerides, avocado oil and mixtures thereof.

4. The water-based matrix according any one of claims 1 to 3, wherein the amount of the at least one vegetable ester of glycerin with one or more mono- and/or polyunsaturated fatty acids, having 18 to 20 carbon atoms, ranges from 3 to 20%, more preferably from 5 to 15% by weight.

5. The water-based matrix according to any one of claims 1 to 4, wherein the at least one non-triglyceride ester formed from an alcohol having 12 to 22 carbon atoms, and one or more saturated, monounsaturated and/or polyunsaturated fatty acids having 14 to 22 carbon atoms is selected from the group consisting of cetearyl olivate, sorbitan olivate, jojoba wax, glyceryl stearate and mixtures thereof.

6. The water-based matrix according to claim 5, wherein the at least one non-triglyceride ester formed from an alcohol having 12 to 22 carbon atoms, and one or more saturated, monounsaturated and/or polyunsaturated fatty acids having 14 to 22 carbon atoms is selected from the group consisting of cetearyl olivate, sorbitan olivate, and mixtures thereof.

7. The water-based matrix according to any one of claims 1 to 6, wherein the amount of the at least one non-triglyceride ester formed from an alcohol having 12 to 22 carbon atoms, and one or more saturated, monounsaturated and/or polyunsaturated fatty acids having 14 to 22 carbon atoms, ranges from 1 to 10%, preferably from 1 to 6%.

8. The water-based matrix according to any one of claims 1 to 7, wherein the at least one polyol of functionality 2 to 12 is selected from the group consisting of glycols, polyalcohols, saccharides and polysaccharides.

9. The water-based matrix according to claim 8, wherein the at least one polyol of functionality 2 to 12 is selected from the group consisting of glycerin, sorbytol and mixtures thereof.

10. The water-based matrix according any one of claims 1 to 9, wherein the amount of the at least one polyol of functionality 2 to 12 ranges from 2 to 6%.

11. The water-based matrix according any one of claims 1 to 10, wherein the at least one sequestrating agent is ethylendiaminetetraacetic acid and/or salts thereof with alkaline metals.

12. The water-based matrix according to claim 11, wherein the at least one sequestrating agent is one or more mono-, bi-, tri- or tetra-sodium salts of ethylendiaminetetraacetic acid.

13. The water-based matrix according any one of claims 1 to 12, wherein the amount of the at least one sequestrating agent is about 0.1 % by weight.

14. The water-based matrix according any one of claims 1 to 13, wherein the at least one antioxidant agent is selected from the group consisting of alphatocopherol and its esters, BHA, esters of gallic acid, nordihydroguaiaracetic acid, idebenone, flavonoids, ascorbic acid, salts and esters of ascorbic acid, carotenoids, tocotrienols and pycnogenol.

15. The water-based matrix according to claim 14, wherein the at least one antioxidant agent is selected from the group consisting of tocopheryl acetate and ascorbyl palmitate, and mixtures thereof.

16. The water-based matrix according any one of claims 1 to 15, wherein the amount of the at least one antioxidant agent is about 3-4%.

17. The water-based matrix according to any one of claims 1 to 16,
wherein the at least one unsaponifiable fraction of a natural oil and/or natural butter containing sterolic fractions of the natural plant from which it derives is an unsaponifiable fraction of vegetable oils and butters selected from the group consisting of shea butter (karite butter), soybean oil, olive oil, wheat germ oil, containing the sterolic fraction of the plant from which it derives.

18. The water-based matrix according any one of claims 1 to 17, wherein the amount of pentylene glycol ranges from 3 to 6%, more preferably it is about 4%.

19. A preservative-free cosmetic formulation comprising a water-based matrix according to any one of claims 1 to 18 and one or more cosmetic active ingredients.

20. The preservative-free cosmetic formulation according to claim 19, wherein the cosmetic active ingredients are anti-wrinkle cosmetic active ingredients in an amount not lower than 1 % with respect to the weight of the final cosmetic formulation, preferably from 1 to 10%.

21. The preservative-free cosmetic formulation according to claim 20, wherein said anti-wrinkle cosmetic active ingredients are one or more cosmetic active ingredients selected from the group consisting of glycerin, hyaluronic acids and its derivatives, retinol and its derivatives, ascorbyl palmitate, tocopheryl acetate, phospholipids and mixtures thereof.

22. The preservative-free cosmetic formulation according to claim 21, wherein the at least one antioxidant agent of the matrix is an anti-wrinkle cosmetic active ingredient helping in reaching the minimum amount of the anti-wrinkle cosmetic active principles.

23. The preservative-free cosmetic formulation according to claim 19, wherein the cosmetic active ingredients are moisturizing cosmetic active ingredients in an amount not lower than 0.5% with respect to the weight of the final cosmetic formulation, preferably 0.5 to 10%.

24. A preservative-free cosmetic formulation according to claim 23, wherein said moisturizing cosmetic active ingredients are one or more cosmetic active ingredients selected from the group consisting of glycerin, hyaluronic acid and its derivatives, and mixtures thereof.

25. The preservative-free cosmetic formulation according to any one of claims 19 to 24, wherein the cosmetic formulation comprises appropriate cosmetic grade excipients with functionality of rheology modifiers, blockers of free water and emulsion stabilizers.

26. The preservative-free cosmetic formulation according to claim 25, wherein the appropriate cosmetic grade excipients are selected from polymeric substances selected from the group consisting of acrylates, cellulose and its derivatives, vinylderivatives, polysaccharides, natural proteic substances and/or their derivatives and hydrolyzed.

27. The preservative-free cosmetic formulation according to claim 25 or claim 26, wherein the amount of the appropriate excipients ranges from 0.003% to 15% with respect to the final weight of the cosmetic formulation.

28. The formulation according to any one of claims 19 to 27, in the form of a cream, a gel, a lypogel or a lotion.

29. The formulation according to claim 28 in the form of a cream.

30. A process of preparing a cream having the formulation according to any one of claims 19 to 27, comprising the steps of:

   a) preparing an aqueous phase and an oil phase with ingredients selected from:

   - at least one polyol of functionality 2 to 12;
   - at least one sequestrating agent,
   - pentylene glycol,
   - at least one non-triglyceride ester formed from an alcohol having 12 to 22 carbon atoms, and one or more saturated, monounsaturated and/or polyunsaturated fatty acids having 14 to 22 carbon atoms,
   - at least one antioxidant agent,
   - at least one unsaponifiable fraction of a natural oil and/or natural butter containing sterolic fractions of the natural plant from which it derives and
   - optionally excipients,
   being each ingredient comprised either in the aqueous phase or in the oil phase based on its nature;

   b) bringing about 50% of the aqueous phase and all the oil phase to a temperature of about 40°C and mixing them until an emulsion is produced;
   c) bringing the resulting emulsion to about 25°C and adding the remaining 50% of the aqueous phase and at least one vegetable ester of glycerin with one or more mono- or polyunsaturated fatty acids, having 18 to 20 carbon atoms;
   d) pasteurizing the resulting emulsion at a temperature ranging from 58°C to 62°C; and
   e) pouring the pasteurized emulsion into sterile containers;

   wherein in either the aqueous phase or the oil phase or in both phases one or more cosmetic active ingredients are present.

31. The process according to claim 30, wherein 50% of the aqueous phase of step b), which must be brought to a temperature of about 40°C, is loaded in a turboemulsifier equipped with temperature and stirring intensity regulation systems.

32. The method according to claim 30 or claim 31, wherein the oil phase is prepared at the emulsifying process of step b) using a "melter".

33. The method according to any one of claims 30 to 32, wherein, in step b), the stirring intensity regulation systems of the thermoemulsifier are blades and turbine, wherein the stirring intensity is adjusted by setting the rotational speed of the turboemulsifier blades at 30 RPM and the one of the turbine at 1000 RPM.

**34.** The method according to any one of claims 30 to 33, wherein, in step c), the resulting emulsion is cooled to 25 °C, by setting a cooling speed of 0.1 °C/min.

**35.** The method according to any one of claims 30 to 34, wherein, in step d), the emulsion is pasteurized at about 60 °C.

Sodium Lauryl Sulphate

Expression of cell viability after treatment with increasing doses of sodium lauryl sulphate

## Figura 1a

L9V9

Expression of cell viability after treatment with different dilutions of the tested product

## Figura 1b

Sodium Lauryl Sulphate

Expression of cell viability after treatment with increasing doses of sodium lauryl sulphate

Figura 2a

L10V10

Expression of cell viability after treatment with different dilutions of the tested product

Figura 2b

Sodium Lauryl Sulphate

Expression of cell viability after treatment with increasing doses of sodium lauryl sulphate

## Figura 3a

L11V11

Expression of cell viability after treatment with different dilutions of the tested product

## Figura 3b

Sodium Lauryl Sulphate

Expression of cell viability after treatment with increasing doses of sodium lauryl sulphate

Figura 4a

L12V12

Expression of cell viability after treatment with different dilutions of the tested product

Figura 4b

## EUROPEAN SEARCH REPORT

European Patent Office

**Application Number**

EP 06 42 5757

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 153 602 A1 (SHISEIDO) 14 November 2001 (2001-11-14) * paragraph [0038] - paragraph [0040] * * paragraph [0043] - paragraph [0044] * * paragraph [0056] - paragraph [0058] * * paragraph [0066] - paragraph [0068] * * examples * | 1-35 | INV. A61K8/34 A61K8/37 A61K8/44 A61K8/67 A61K8/92 A61K8/97 A61Q19/00 A61Q19/08 |
| Y | US 5 741 496 A (YVES ROCHER) 21 April 1998 (1998-04-21) * column 2, line 20 - line 65 * * column 3, line 35 - line 45 * | 1-35 | |
| Y | EP 1 275 371 A (JOHNSON AND JOHNSON) 15 January 2003 (2003-01-15) * paragraph [0066] - paragraph [0082] * * paragraph [0101] - paragraph [0110] * * paragraph [0129] - paragraph [0130] * | 1-35 | |
| Y | WO 2004/006869 A (JOHNSON & JOHNSON) 22 January 2004 (2004-01-22) * page 14, line 27 - page 18, line 14 * * page 21, line 27 - page 22, line 14 * * page 28, line 20 - line 24 * * examples * | 1-35 | TECHNICAL FIELDS SEARCHED (IPC) A61K |
| Y | US 4 454 159 A (MUSHER, ALBERT) 12 June 1984 (1984-06-12) * column 1, line 52 - column 2, line 22 * * column 3, line 1 - line 40 * | 1-35 | |

The present search report has been drawn up for all claims

| Place of search | Date of completion of the search | Examiner |
|---|---|---|
| Munich | 29 May 2007 | Irwin, Lucy |

## ANNEX TO THE EUROPEAN SEARCH REPORT
## ON EUROPEAN PATENT APPLICATION NO.

EP 06 42 5757

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2007

| Patent document cited in search report | | Publication date | Patent family member(s) | | Publication date |
|---|---|---|---|---|---|
| EP 1153602 | A1 | 14-11-2001 | WO<br>TW<br>US | 0145665 A1<br>231218 B<br>2003091603 A1 | 28-06-2001<br>21-04-2005<br>15-05-2003 |
| US 5741496 | A | 21-04-1998 | NONE | | |
| EP 1275371 | A | 15-01-2003 | AT<br>BR<br>CA<br>CN<br>WO<br>JP<br>MX<br>US | 353006 T<br>0211222 A<br>2456062 A1<br>1527695 A<br>03005983 A1<br>2005508452 T<br>PA04000361 A<br>2005048856 A1 | 15-02-2007<br>10-08-2004<br>23-01-2003<br>08-09-2004<br>23-01-2003<br>31-03-2005<br>07-09-2005<br>03-03-2005 |
| WO 2004006869 | A | 22-01-2004 | AU<br>BR<br>CA<br>CN<br>JP<br>KR<br>US | 2003250010 A1<br>0305505 A<br>2492280 A1<br>1674856 A<br>2006504649 T<br>20050049468 A<br>2006159924 A1 | 02-02-2004<br>28-09-2004<br>22-01-2004<br>28-09-2005<br>09-02-2006<br>25-05-2005<br>20-07-2006 |
| US 4454159 | A | 12-06-1984 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GAWKRODGER DJ.** Nickel dermatitis: how much Nickel is safe. *Contact Dermatitis,* 1996, vol. 35, 267-271

- **TANOJO H ; HOSTYNEK JJ ; MOUNTFORD HB ; MIBACH HI.** In vitro premeation of nickel salts through human stratum corneum. *Acta Derm Venereol Suppl,* 2001, vol. 212, 19-23